Europäisches Patentamt

European Patent Office

Office européen des brevets

(11) Veröffentlichungsnummer: **0 417 407 B1**

(12) **EUROPÄISCHE PATENTSCHRIFT**

(45) Veröffentlichungstag der Patentschrift: **11.05.94**

(21) Anmeldenummer: **90111500.6**

(22) Anmeldetag: **19.06.90**

Die Akte enthält technische Angaben, die nach dem Eingang der Anmeldung eingereicht wurden und die nicht in dieser Patentschrift enthalten sind.

(51) Int. Cl.⁵: **B01J 31/08**, B01J 31/10, B01J 47/00, C07C 29/04

(54) **Formkörper aus makroporösen Ionenaustauscherharzen sowie Verwendung der Formkörper.**

(30) Priorität: **13.09.89 DE 3930515**

(43) Veröffentlichungstag der Anmeldung:
**20.03.91 Patentblatt 91/12**

(45) Bekanntmachung des Hinweises auf die Patenterteilung:
**11.05.94 Patentblatt 94/19**

(84) Benannte Vertragsstaaten:
**AT BE CH DE DK ES FR GB GR IT LI LU NL SE**

(56) Entgegenhaltungen:
**EP-A- 0 018 511**
**DE-B- 1 285 170**
**FR-A- 2 064 778**
**FR-A- 2 297 083**
**FR-A- 2 398 711**

(73) Patentinhaber: **VEBA OEL AG**
**Alexander-von-Humboldt-Strasse**
**D-45896 Gelsenkirchen(DE)**

(72) Erfinder: **Gottlieb, Klaus, Dr.**
**Alte Strasse 59**
**D-5804 Herdecke(DE)**
Erfinder: **Graf, Wilfried**
**Lütkenbusch 4**
**D-4270 Dorsten(DE)**
Erfinder: **Schädlich, Kuno, Dr.**
**Am Krausen Bäumchen 129**
**D-4300 Essen(DE)**
Erfinder: **Hoffmann, Ulrich, Prof. Dr.**
**Hans-Sommer-Strasse 10**
**D-3300 Braunschweig(DE)**
Erfinder: **Rehfinger, Alwin, Dr. Ing.**
**Rosenstrasse 10**
**D-6704 Mutterstadt(DE)**
Erfinder: **Flato, Jörg, Dipl.-Ing.**
**Am Breiten Busch 17**
**D-3360 Osterrode(DE)**

(74) Vertreter: **Lindner, Wolfgang, Dr.**
**Alexander-von-Humboldt-Strasse**
**D-45896 Gelsenkirchen (DE)**

**Beschreibung**

Die Erfindung betrifft Formkörper aus makroporösem Ionenaustauscherharz sowie die Verwendung solcher Formkörper.

Makroporöse Ionenaustauscherharze sind als Packungsmaterial beispielsweise für Chromatographiesäulen bekannt. So ist in der Druckschrift EP 0 104 912-A die Auftrennung von Zuckern mittels Flüssigchromatographie über eine mit porösem sulfonsaurem Divinylbenzol-Styrol-Copolymerisat gefüllte Säule beschrieben. Das Packungsmaterial liegt in Partikel Größen von 1 bis 30 $\mu$m vor.

In der Dissertation von A. Rehfinger, Technische Universität Clausthal, 1988, wurde die katalytische Veretherung von Isobuten und Methanol zu Methyl-tert.-butylether (MTBE) an makroporösem starksaurem Ionenainstauscherharz als Katalysator einer erneuten eingehenden reaktionstechnischen Untersuchung unterzogen. Der eingesetzte Katalysator lag in Partikelgrößen von 0,3 bis 1,2 mm vor.

In dem Dokument JP-A-58 133 821 ist bei einem Verfahren zur Durchführung eines Austausches des Wasserstoffisotops Deinterium durch Wasserstoff in einer Gleichgewichtsreaktion zwischen gasförmigem Wasserstoff und Wasserdampf ein mit Platin beladener hydrophober Katalysator aus einem Styrol-Divinylbenzol-Copolymer o. dgl. als Träger, in Form von Kügelchen, Pellets, gewebten oder gewirkten Flächengebilden, Raschig-Ringen, oder dgl., eingesetzt. Der Katalysator ist in ein Netzwerk aus Edelstahl oder Kunststoffharz, das den Durchgang von flüssigem Wasser behindert, eingebracht.

In dem Abschlußbericht von R. P. Arganbright, Dennis Hearn, Edward M. Jones, Lawrence Smith, DOE/CS/40 454-T3, September 1986, der Chemical Research and Licensing Company and Neochem Corporation wird ein neues Verfahren zur Herstellung von MTBE mittels katalytischer Destillation beschrieben, das die gleichzeitige Katalyse und Destillation in einem Reaktionsapparat erlaubt. Als Katalysator wurde sulfonsaures Divinylbenzol-Styrol-Copolymerisat in Partikelgrößen von 0,1 bis 0,5 mm, welches in durch Abnähen entstandene Taschen gürtelförmiger Streifen eines Fiberglasgewebes eingebracht ist, verwendet. Nach dem Füllen der Taschen werden deren Öffnungen durch Abnähen ebenfalls verschlossen und die Gürtel mit zwischengelegten Schichten einer Maschendrahtstruktur aus Edelstahl mit einer Maschenweite von 12 in. zu zylinderischen Körpern zwecks Einsatz in eine Festbett-Reaktor-Kolonne aufgerollt.

Diese Methoden haben jedoch den Nachteil, daß sich der Stofftransport durch die Umhüllung des eigentlichen Ionenaustauschers erheblich verschlechtert.

Die Aufgabe der vorliegenden Erfindung besteht nun darin, die Kombination einer chemischen Reaktion mit der gleichzeitigen stofflichen Trennung des Reaktionsgemisches in einem Apparat zu erreichen, wobei ein in diesem Apparat angeordneter katalytisch aktiver Füllkörper die Reaktion beschleunigt und der für die Trennung erforderliche intensive Stoffaustausch ermöglicht wird.

Diese Aufgabe wird durch die Formkörper gemäß den Ansprüchen 1 bis 5 und deren Verwendung gemäß den Ansprüchen 6 bis 8 gelöst.

Die Formkörper bestehen aus starksaurem, schwachsaurem oder basischem makroporösem Ionenaustauscherharz oder auch aus gelartigen Harzen in Gestalt von Trennkörperformen, d. h. Formen von Füllkörpern, die in der chemischen Technik durch Oberflächen-Vergrößerung zwischen gas- oder dampfförmigen Stoffen oder auch Flüssigkeiten im Gegen- oder Gleichstrom der Reaktionsbeschleunigung dienen, beispielsweise Raschig-Ringen, Berl-Sätteln, Torus-Sätteln, Füllringen mit Steg oder Kreuzsteg, Pall-Ringen, sonstigen Hohlkörpern, Hohlkugeln oder dgl.

Die Formkörper werden, bezogen auf die Makroform ohne Poren, mit einem Hohlraumanteil von 50 bis 95 Vol.-%, einer BET-Oberfläche von 0,01 bis 1 000, vorzugsweise 20 bis 60 $m^2$/g und einer Austauschkapazität von 0,05 bis 10, vorzugsweise 3 bis 6 meq/g hergestellt und eingesetzt. Die Messung der BET-Oberfläche erfolgte nach dem Einpunkt-Differenzverfahren nach Haul-Dümbgen (DIN 66132).

Die Formkörper werden zweckmäßig unter Anwendung einer Polymerisationsinitiierung, beispielsweise durch Zusatz eines Radikale liefernden Initiators, und durch Copolymerisation aus Styrol und Divinylbenzol im Gewichtsverhältnis von 200:1 bis 1:8, vorzugsweise von 20:1 bis 5:1, gegebenenfalls unter Zugabe von 2 bis 80, vorzugsweise 10 bis 40 Gew.-% Porenbildner zu der Gesamtmischung hergestellt.

Eine wichtige Gruppe von Formkörpern wird aus starksaurem makroporösem Ionenaustauscherharz durch Generierung oder Aufbringung saurer Zentren, insbesondere nachträgliche Säurebehandlung, z. B. mit Sulfonsäure, hergestellt.

Für bestimmte Reaktionen ist es zweckmäßig, Formkörper der vorstehend beschriebenen Art mit Metallen der 7. oder auch 8. Nebengruppe des Periodensystems, insbesondere Palladium, Platin, Ruthenium oder Rhodium in Mengen von 0,1 bis 100 g/l Ionenaustauscherharz zu dotieren.

Eine zweckmäßige äußere Gestaltung der vorstehend beschriebenen Formkörper besteht in der Form von Raschig-Ringen mit Innendurchmessern von 0,5 bis 100 mm, vorzugsweise 4 bis 20 mm, Wandstärken von 0,1 bis 20 mm, vorzugsweise 0,5 bis 3 mm und einer Länge, die dem 0,1- bis 20-fachen des Innendurchmessers entspricht.

Die Erfindung besteht ferner in der Verwendung der vorstehend definierten Formkörper als katalytisch aktive Füllkörper für chemische Reaktionen oder Kombinationen derselben, insbesondere Veretherung, Hydratisierung, Dimerisierung, Oligomerisierung, Veresterung, Hydrierung oder Alkylierung.

Besonders bevorzugt ist die Verwendung der Formkörper mit gleichzeitig angewandter Trennoperation wie Adsorption, Absorption, Extraktion, Strippen, Destillation, Rektifikation, Fraktionierung, Membranverfahren od. dgl.

Durch die Herstellung und Verwendung eines Ionenaustauscherharzes als Formkörper ist es möglich geworden, den Ionenaustauscher gleichzeitig als Füllkörper zur Schaffung einer Phasengrenzfläche und als Katalysator in einer Füllkörper-Reaktions-Kolonne einzusetzen. Die Herstellung der neuen Formkörper, beispielsweise mit Abmessungen die einem herkömmlichen 7 mm-Raschigring mit einer Wandstärke von 1 mm entsprechen, ermöglicht erstmals den für eine Gegenstromführung von Gas- und Flüssigphase erforderlichen Lückengrad einer Schüttung von Ionenaustauschermaterial.

Weiterhin wirkt sich die - gegenüber früheren Versuchen zur Überführung einzelner Kügelchen des Ionenaustauschers in größere Einheiten mit entsprechendem Lückengrad durch Verbinden einzelner Kügelchen - durch die erfindungsgemäßen Formkörper ermöglichte gleichmäßige Materialstärke vorteilhaft für eine gezielte Reaktionsführung aus.

Als Ionenaustauscherharze kommen insbesondere Harze aus Divinylbenzol-Styrol-Copolymerisaten, aber auch Phenol-Formaldehydharze, Harnstoffharze oder Kondensationsprodukte von aromatischen Diaminen mit Formaldehyd in Betracht.

Die Herstellung der Formkörper kann durch Blockpolymerisation des Monomergemisches in entsprechenden Gießformen erfolgen, beispielsweise durch Ringspaltpolymerisation in Teflongießformen. Auch andere Vorrichtungen, beispielsweise Extruder oder Spritzgußmaschinen, sowie sonstige dem Fachmann zur Verfügung stehende Vorrichtungen zur Formgebung aushärtender Polymerisationsansätze sind geeignet.

Zur Bildung der Formkörper sind insbesondere auch gelartige Harze geeignet, welche in den geeigneten Vorrichtungen zu den gewünschten Füllkörperformen verarbeitet werden können. So ist es auch möglich, noch nicht vollständig ausgehärtete, noch formbare Harze in entsprechende Füllkörperformen zu bringen, um durch Anwendung geeigneter Bedingungen nach Formgebung eine endgültige Aushärtung zwecks Erhalts der notwendigen Festigkeiten für die Verwendung als Füllkörperpackungen in verfahrenstechnischen Apparaten zu gewährleisten.

Durch Zugabe inerter Porenbildner wie Alkane, beispielsweise n-Pentadecan, im angegebenen Mengenverhältnis gelingt es, die gewünschte makroporöse Struktur entsprechend den gewünschten Spezifikationen herzustellen.

Als weitere Hilfsstoffe können Polymerisationinitiatoren wie z. B. Azoisobuttersäurenitril (AIBN) oder andere eingesetzt werden.

Bei den starksauren makroporösen Inonenaustauscherharzen aus mit Divinylbenzol vernetztem Polystyrol sind als Ladungsträger die an der Polymermatrix durch Sulfonierung verankerten $SO_3H$-Gruppen für die Austauschkapazität und auch die katalytische Aktivität verantwortlich.

Durch den makroporösen Aufbau werden die angegebenen Werte der inneren Oberfläche erreicht, die herkömmlichen, im Handel befindlichen makroporösen Typen entsprechen.

Nachfolgend wird die Herstellung eines speziellen makroporösen starksauren Harzes aus Divinylbenzol-Styrol-Copolymerisat in Form von Raschig-Ringen beschrieben.

Beispiel 1:

Es wird eine Monomermischung aus Styrol und Divinylbenzol im Gew.-Verhältnis von 13:1 angesetzt. Nach Zugabe von n-Pentadecan als inerter Porenbildner in einer Menge von 35 Gew.-% und einer wirksamen Menge von AIBN als Polymerisationsinitiator ist die Mischung zum Einfüllen in die Gießform fertig.

Als Gießform dient ein Ringspalt aus innen- und außenverstärkten Teflonschläuchen. Zwei Teflonringsegmente dienen zur Abdichtung und als Abstandshalter. Der Ringspalt wies einen Innendurchmesser von 4 mm bei einer Wandstärke von 0,5 mmm auf.

Die Mischung wurde 5 Stunden bei 80 °C ausreagieren gelassen. Danach wurden die Rohlinge aus der Form gezogen und auf Länge abgesägt. Zur Entfernung des n-Pentadecans aus den gebildeten Formkörpern wurde eine erste Spülung mit Chloroform durchgeführt.

Zur Sulfonierung wurden 350 ml der nach dem Vorstehenden hergestellten Raschig-Ringe mit 600 ml Chloroform und 80 ml Chlorsulfonsäure in einem Rundkolben im Eisbad und unter Rückflußkühlung unter gelegentlichem Rühren für 20 Stunden ausreagieren gelassen. Im Anschluß hieran wurden weitere 40 ml Chlorsulfonsäure zugegeben und weitere 3 bis 5 Stunden bei Raumtemperatur reagieren gelassen.

Die sulfonierten Ringe wurden zunächst mit Chloroform, dann mit Methanol und schließlich mit Wasser gespült, bis ein pH-Wert von mindestens 3,0 erreicht war. Die Lagerung der Ringe erfolgte in destilliertem Wasser.

Die Belegung der starksauren Formkörper aus makroporösen starksauren Harzen mit einem oder mehreren Metallen der 7. oder auch 8. Nebengruppe des periodischen Systems der Elemente kann beispielsweise so erfolgen, daß man zunächst die Formkörper mit einer Lösung der gewünschten Metalle, beispielsweise mit einer wäßrigen Lösung nicht-komplexer, kationischer Salze, behandelt. Solche Metallsalze können beispielsweise Chloride, Bromide, Nitrate, Sulfate und/oder Acetate sein. Die Menge des Salzes wird so gewählt, daß sich nach der weiteren Behandlung, insbesondere nach der Reduzierung, die gewünschte Menge Metall auf den Formkörpern befindet. Nach der Behandlung mit Metallsalzlösung werden die Formkörper mit Wasser neutralgewaschen und getrocknet, beispielsweise bei erhöhter Temperatur und ggf. bei vermindertem Druck. Die Überführung der aufgebrachten Metalle in den elementaren Zustand erfolgt durch Behandlung mit Reduktionsmitteln, z. B. Wasserstoff, beispielsweise bei einem Druck von 2 bis 50, vorzugsweise 20 bis 30 bar, und einer Temperatur von 50 bis 140, vorzugsweise 80 bis 120 °C.

Die Formkörper sind für die Verwendung als katalytisch aktive Füllkörper für einphasige und mehrphasige Gas- und Flüssigreaktionen, insbesondere bei Gegenstromführung der Phasen, geeignet, da sie einen hohen Lückengrad aufweisen und somit einen niedrigen Druckverlust verursachen.

Die Formkörper sind besonders geeignet für die Verwendung als katalytisch aktive Füllkörper zur Durchführung sogenannter katalytischer Destillationen, bei denen die Reaktion und die sich üblicherweise in einem weiteren Verfahrensschritt anschließende Aufarbeitung der Reaktionsprodukte durch Destillation bzw. Rektifikation simultan in einem Reaktionsapparat erfolgen.

Der Einbau der Formkörper in den Reaktor erfolgt zweckmäßig durch Einbringen loser Schüttungen auf Tragroste. Reaktoreingang und Reaktorausgang können mit Siebgewebe versehen werden, um kleinere Partikel, Bruchstücke, Abrieb o. dgl. zurückzuhalten bzw. den Überriß solcher Teilchen zu vermeiden. Zur Minderung der mechanischen Belastung können die Formkörper auf mehreren übereinander angeordneten Tragrosten angeordnet werden.

Besonders geeignet für diese Art der Reaktionsführung sind chemische Reaktionen, bei denen die Siedepunkte der Reaktanden und des Produktes verschieden sind.

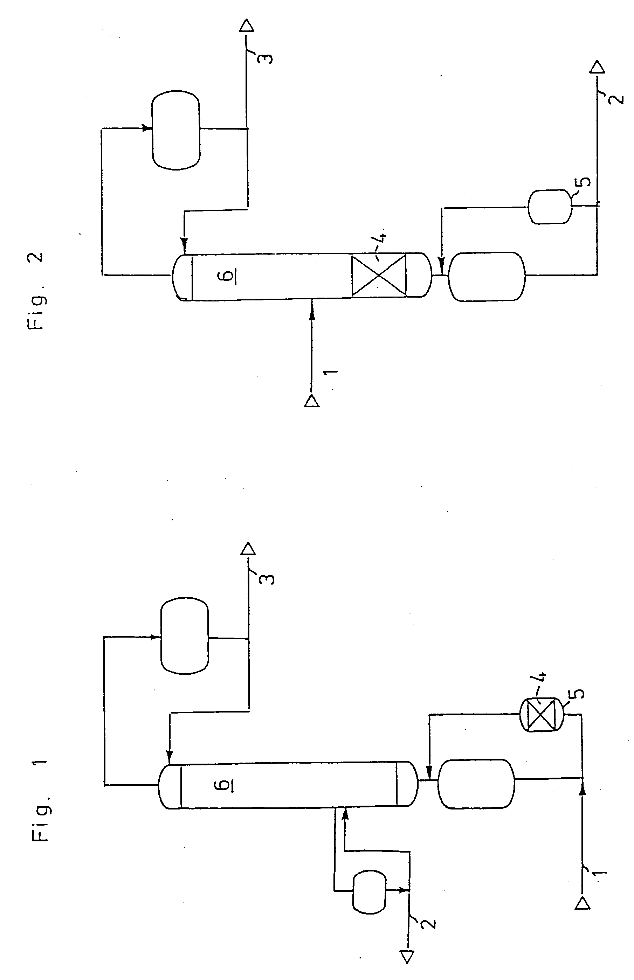

Es sind beispielsweise folgende Anwendungsfälle gemäß den Ausführungsbeispielen, wie sie in den Schemata der Figuren 1 bis 3 skizziert sind, von Interesse:

1. Die entstehenden Reaktionsprodukte sind in der Siedelage niedriger als das Einsatzgemisch.

Das Einsatzgemisch gelangt über Leitung 1 in den Aufkocher 5, der mit Formkörpern 4 gefüllt ist. Die entstehenden Reaktionsprodukte werden entsprechend ihrer Siedelage in der Rektifiziersäule 6 aufgetrennt und über Leitung 2 und 3 abgeführt (Fig. 1).

2. Die entstehenden Produkte sind in der Siedelage höher und niedriger als das Einsatzgemisch.

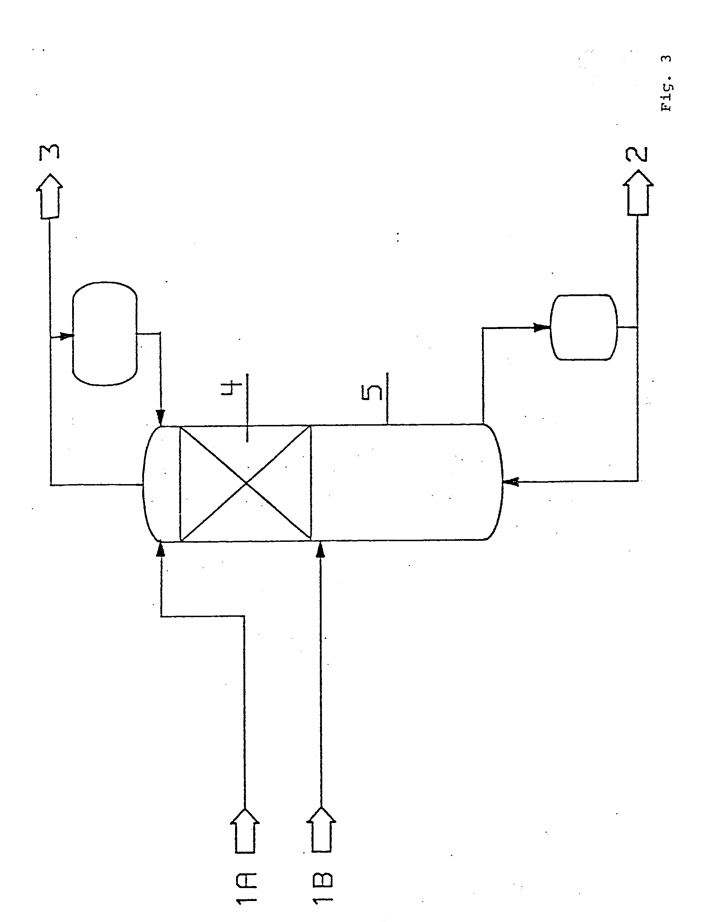

Das Einsatzgemisch wird über Leitung 1 oberhalb der Formkörperschüttung 4 eingebracht und in der Schüttung in eine leichter und eine schwerer siedende Komponente umgesetzt. Am Kopf der Rektifiziersäule 6 wird die leichter siedende Komponente über Leitung 3 und die schwerer siedende Komponente aus dem Sumpf über Leitung 2 abgezogen. (Fig. 2)

3. Das in Fig. 3 dargestellte Beispiel betrifft den Fall, daß der Strom eines Einsatzgemisches 1B aus mehreren Komponenten besteht, die sich in der Siedelage nur unwesentlich voneinander unterscheiden. In seiner Gesamtheit gesehen liegt seine Siedetemperatur jedoch am niedrigsten. Der Strom des Einsatzgemisches 1A stellt nur eine einzige Komponente dar und liegt in seiner Siedetemperatur knapp über der Siedetemperatur des Reaktionsproduktes 2, aber wesentlich höher als der Strom des Einsatzgemisches 1B bzw. des Kopfproduktes 3.

Der schwerer siedende Strom des Einsatzgemisches 1A wird am Kopf, oberhalb der Katalysator-Ring-Schüttung 4, der leichter siedende Strom in der Mitte unterhalb der Schüttung in eine Rektifiziersäule 5 gegeben. Entsprechend seiner Siedelage sind die Komponenten des Stoffstroms 1A bestrebt, sich in der unteren Hälfte der Säule, und die Komponenten des Stoffstroms 1B, sich am Kopf der Säule anzureichern. Auf dem Weg dorthin reagieren die Komponenten von 1A jedoch mit einer Komponente von 1B hochselektiv zu dem schwer siedenden Produktstrom 2, der im Sumpf abgezogen werden kann. Das um eine Komponente verminderte Destillat 3 kann nachfolgenden Prozessen zugeführt werden (Fig. 3).

Die "katalytische Destillation" bietet neben dem Vorteil, daß eine Verfahrensstufe eingespart wird und somit der apparative Aufwand vermindert wird, auch Vorteile hinsichtlich der Verfahrensführung. Dieses bevorzugt für exotherme Reaktionen in Frage kommende Verfahren ermöglicht die unmittelbare Ausnutzung der freiwerdenden Reaktionswärme zur gleichzeitigen Destillation der Reaktionsprodukte. Die Reaktionstemperatur wird so vorgegeben, daß sie dem Siedepunkt des Produktes entspricht, wodurch sich eine einfache Temperaturführung ergibt.

Das gebildete Reaktionsprodukt wird durch die simultane Destillation der Reaktionszone sofort entzogen, wodurch die Bildung von Folge- oder auch Parallelprodukten vermindert wird. Durch das sofortige Entfernen der Reaktionsprodukte können Gleichgewichtsreaktionen nahezu vollständig ablaufen. Entsprechend der Gleichgewichtskonstanten wird sofort neues Reaktionsprodukt gebildet.

Reaktionen, die nach diesem Prinzip der katalytischen Destillation durchgeführt werden können, sind exotherme Reaktionen wie die Veretherung, beispielsweise die Herstellung von Methyl-tert.-butylether (MTBE) aus Methanol und Isobuten, die Hydratisierung, beispielsweise die Herstellung von Isopropanol aus Propen und Wasser, die Dimerisierung, beispielsweise von Isobuten zu Di-isobuten, die Veresterung, beispielsweise von Essigsäure mit Isopropanol zu Isopropylacetat, die Alkylierung, beispielsweise von Phenol und Propen zu Propylphenol und die Oligomerisierung, beispielsweise von Butenen zu wertvollen Komponenten sogenannter Alkylatbenzine.

Die Formkörper sind als bifunktionell katalytisch aktive Füllkörper für katalytische Reaktionen der Hydratisierung und Veretherung mit gleichzeitiger Hydrierung geeignet. Ein Beispiel hierfür ist die Veretherung von Pyrolyseleichtbenzin zu tert.-Amylmethylether mit gleichzeitiger selektiver Hydrierung von Dienen, z. B. von Cyclopentadien und dessen Derivaten zu den entsprechenden Monoolefinen.

Auch die Zersetzung von MTBE in Isobuten und Methanol kann nach dem Verfahrensprinzip der katalytischen Destillation erfolgen. Hieraus ergibt sich ein Weg zur Gewinnung hochreiner Produkte, im vorliegenden Fall von hochreinem Isobuten. Ein weiterer Anwendungsfall ist die Abtrennung von Isoamylen aus einem C5-Schnitt, indem zunächst ein tertiärer Amylalkylether mittels katalytischer Destillation gebildet wird und dieser in einer zweiten katalytischen Destillation in hochreines Isoamylen und den Alkohol zersetzt wird.

Hinsichtlich des Einsatzes als Packungsmaterial für katalytische Destillationen weisen die erfindungsgemäßen Formkörper gegenüber handelsüblichem kleinem kugelförmigem Ionenaustauschermaterial aus Divinylbenzol-Styrol-Copolymerisat entscheidende Vorteile auf. Sie dienen gleichzeitig als Füllkörper zur Schaffung einer Phasengrenzfläche und als Katalysator. Ermöglicht wird ein solcher Simultanbetrieb durch den geringen Druckverlust der Formkörper.

Reaktionstechnische Untersuchungen haben gezeigt, daß die erfindungsgemäßen Formkörper die gleiche Aktivität aufweisen wie vergleichbare herkömmliche Katalysatoren. Darüberhinaus wird ein besonderer Vorteil darin gesehen, ein katalytisch aktives Ionenaustauscherharz in Gestalt von Formkörpern zur Verfügung zu stellen, die aufgrund ihres hohen Lückengrades den Einsatz als Füllkörper ermöglichen und einen bestmöglichen Wärme- und Stoffaustausch zwischen den beteiligten Fluidphasen gewährleisten.

Die nachfolgenden Vergleichsversuchsergebnisse verdeutlichen am Beispiel der MTBE-Synthese, daß die Formkörper aus Ionenaustauscherharz über eine vergleichbare katalytische Aktivität verfügen (Beispiel 2) wie ein handelsüblicher Katalysator (hier Amberlyst (R) 15), der in Form von Partikeln mit einem durchschnittlichen Durchmesser von 0,2 bis 2 mm vorlag (Vergleichsbeispiel 3).

Beispiel 2:

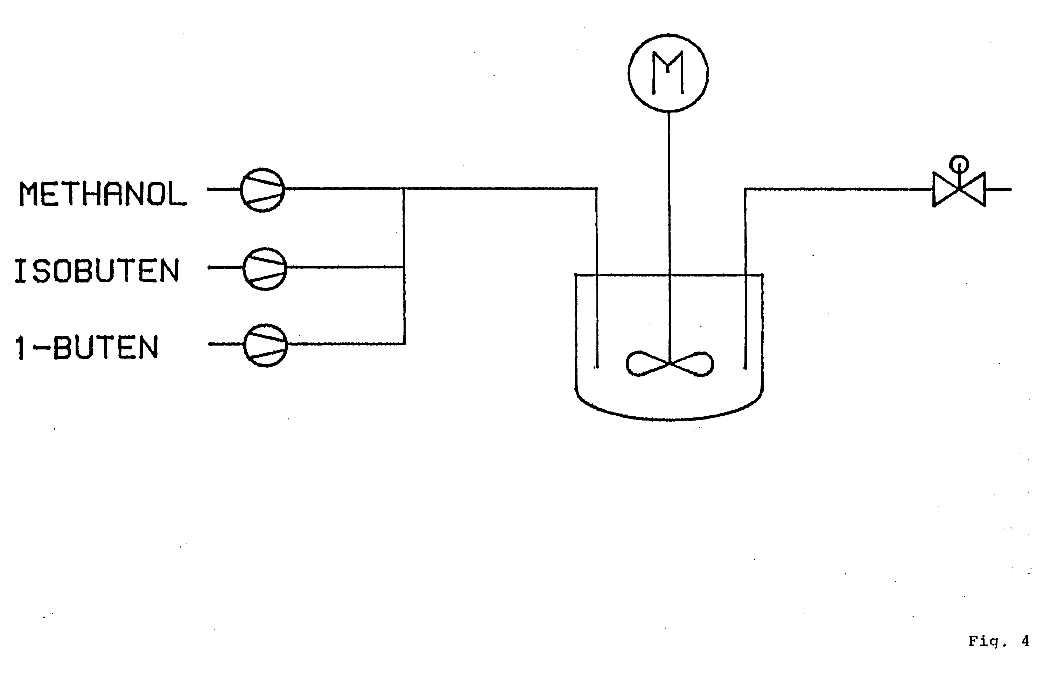

Zur Untersuchung der Reaktionskinetik der MTBE-Synthese werden die Reaktanden Methanol und Isobuten nebst dem Verdünnungsmedium 1-Buten über Dosierpumpen in einen Rührkessel von 106 ml Inhalt gefördert. Der Volumenstrom wurde so gewählt, daß sich eine mittlere Verweilzeit von 5,3 min im Reaktor einstellt. Eine Prinzipskizze zeigt Fig. 4.

Die Katalysatorringe wurden im Reaktor auf ein fixiertes Teflonröhrchen aufgezogen.

Die Probenahme erfolgte über einen Teilstrom des Ablaufgemisches, der kontinuierlich durch eine Probenschleife geführt wurde.

Die erhaltenen Ergebnisse bei den angewandten Parametern sind in der nachstehenden Aufstellung wiedergegeben.

| Nr. | Konzentrationen in Gew.% | | | | | $U_{MeOH}$ in % |
|-----|------|------|------|------|------|------|
| | MeOH | IB | 1B | MTBE | DIB | |
| 1 | 42,7 | 55,7 | – | 1,63 | – | 1,37 |
| 2 | 30,8 | 57,8 | 9,43 | 1,97 | – | 2,27 |
| 3 | 16,9 | 60,5 | 20,0 | 2,51 | 0,088 | 5,12 |
| 4 | 5,96 | 62,5 | 29,6 | 1,40 | 0,507 | 7,86 |
| 5 | 4,53 | 63,1 | 30,4 | 1,41 | 0,556 | 10,2 |
| 6 | 3,03 | 63,2 | 31,5 | 1,53 | 0,740 | 15,5 |

Reaktionsgeschwindigkeiten
in mmol/s eq

| Nr. | DIB | MTBE |
|-----|------|------|
| 1 | – | 16,8 |
| 2 | – | 19,5 |
| 3 | 0,66 | 23,9 |
| 4 | 8,45 | 29,7 |
| 5 | 9,24 | 29,8 |
| 6 | 12,2 | 32,3 |

Abkürzungen: MeOH – Methanol

IB – Isobuten

MTBE – Methyl-tert.-butylether

DIB – Diisobuten

1B – 1-Buten

Reaktionsgleichungen: MEOH + IB $\rightarrow$ MTBE

IB + IB $\rightarrow$ DIB

Reaktionsbedingungen: T = 60 °C, p = 21 bar.

6

Katalysatorschüttung: 1,0 g Raschig-Ringe aus starksaurem makroporösem Divinylbenzol-Styrol-Copolymerisat, Maße (in Wasser gequollen) : 6 x 6 x 1 mm, Aufquellung (trocken zu Wasser) : 80 Vol.-%, Hohlraumanteil ca. 45 Vol.-%, Vernetzungsgrad: 7,5 Gew.-% Divinylbenzol in Monomermischung mit Styrol, BET-Oberfläche (1 Punkt -Methode, $N_2$-Adsorption) 30 $m^2$/g, Austauschkapazität 4,5 meq/g.

Die ermittelte Anfangsreaktionsgeschwindigkeit ist für die patentgegenständlichen Katalysator-Ringe praktisch genauso groß wie für unter vergleichbaren Bedingungen eingesetztes Amberlyst (R) 15, wie der Vergleich mit dem folgenden Beispiel 3 zeigt.

Beispiel 3 (Vergleichsbeispiel)

Die nachstehenden Versuchsergebnisse wurden unter den gleichen Bedingungen wie bei Beispiel 2 erhalten mit dem einzigen Unterschied, daß der Katalysator nun in Kugelform vorlag und nicht mehr im Reaktor fixiert war, sondern durch den Rührer in Schwebe gehalten wurde. Die eingewogene Katalysatormenge betrug ebenfalls 1,0 g.

| Nr. | Konzentrationen in Gew.% | | | | | $U_{MeOH}$ in % |
|---|---|---|---|---|---|---|
| | MeOH | IB | 1B | MTBE | DIB | |
| 1 | 39,2 | 55,0 | 5,22 | 0,544 | - | 0,502 |
| 2 | 22,0 | 58,0 | 19,4 | 0,549 | - | 0,898 |
| 3 | 9,2 | 61,0 | 28,7 | 1,02 | 0,097 | 3,870 |
| 4 | 4,25 | 60,3 | 33,2 | 1,88 | 0,295 | 13,800 |
| 5 | 2,72 | 60,2 | 34,3 | 2,34 | 0,474 | 23,800 |

| Nr. | Reaktionsgeschwindigkeiten in mmol/s eq | |
|---|---|---|
| | DIB | MTBE |
| 1 | - | 18,2 |
| 2 | - | 17,5 |
| 3 | 2,30 | 31,3 |
| 4 | 6,18 | 56,8 |
| 5 | 9,21 | 70,6 |

Beispiel 4:

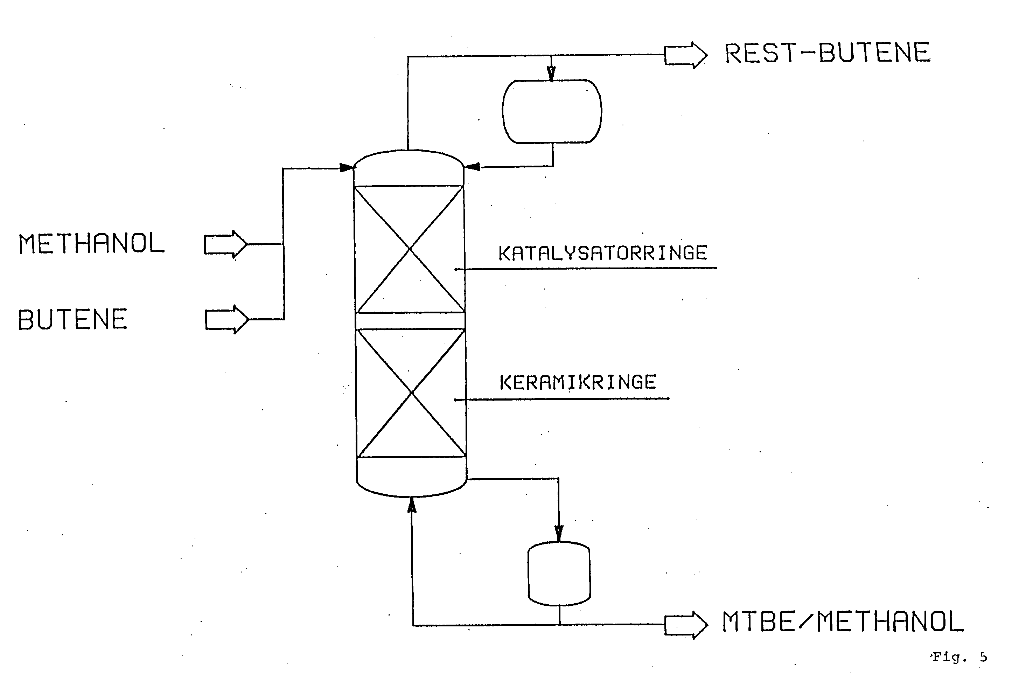

In weitergehenden Versuchen wurden die erfindungsgegenständlichen Katalysatorringe in einer aus Edelstahl (Wst.Nr. 1,4571) gefertigten Füllkörperkolonne eingesetzt. Die Reaktanden Methanol und Isobuten wurden nebst dem Verdünnungsmedium 1-Buten über Dosierpumpen am Kopf der Kolonne eingespeist. Das Gemisch wird annähernd auf Siedetemperatur aufgeheizt. Die obere Hälfte der Trennsäule ist mit den chemisch aktiven Formkörpern in Form von Katalysator-Ringen (Raschig-Ringen) gefüllt, während die untere Hälfte mit inerten Keramik-Ringen gleicher Abmessung gefüllt ist (vgl. Fig. 5). Die Kolonne hatte einen Innendurchmesser von 53 mm. Die Gesamtschütthöhe der Füllkörper betrug 1 m. Zur Minderung der mechanischen Belastung wurden die Katalysator-Ringe von drei übereinander geordneten Tragrosten gehalten.

Die Probenahme erfolgte über einen Teilstrom des Kopf- bzw. Sumpfproduktes, welcher kontinuierlich durch Probenschleifen geführt wurde.

In den nachfolgenden Tabellen sind die Ergebnisse einiger Versuchsläufe 1 bis 9 in der vorher beschriebenen Apparatur mit den erfindungsgegenständlichen Katalysator-Ringen dargestellt, wobei der Stoffstrom für die Einsatzprodukte einen Wert von 25 ml/min bei einem Anlagendruck von 6 bar (absolut) und einer Temperatur unterhalb der Katalysatorschüttung von 80 °C aufwies.

| Nr. | Molare Zusammensetzung des Feeds in % | | |
|---|---|---|---|
| | $X_{MeOH}$ | $X_{1B}$ | $X_{IB}$ |
| 1 | 25,0 | 63,75 | 11,25 |
| 2 | 25,0 | 50,5 | 24,5 |
| 3 | 25,0 | 37,5 | 37,5 |
| 4 | 52,5 | 23,5 | 24,0 |
| 5 | 52,5 | 32,0 | 15,5 |
| 6 | 52,5 | 40,5 | 7,0 |
| 7 | 80,0 | 10,0 | 10,0 |
| 8 | 80,0 | 13,5 | 6,5 |
| 9 | 80,0 | 17,0 | 3,0 |

| Molare Zusammensetzung des Sumpfproduktes (%) | | | | | | | |
|---|---|---|---|---|---|---|---|
| Nr. | $X_{MTBE}$ | $X_{MeOH}$ | $X_{DI}$ | $X_{\beta DI}$ | $X_{Tri}$ | $X_{\beta Tri}$ | IB Umsatz |
| 1 | 73,08 | 26,92 | Spuren | 0 | 0 | 0 | 58,79 |
| 2 | 84,01 | 15,99 | Spuren | Spuren | 0 | 0 | 70,07 |
| 3 | 67,78 | 14,48 | 12,83 | 4,305 | 0,28 | 0,326 | 79,02 |
| 4 | 44,75 | 55,25 | Spuren | Spuren | Spuren | Spuren | 71,65 |
| 5 | 22,94 | 76,89 | 0,085 | 0,083 | Spuren | Spuren | 69,40 |
| 6 | 9,29 | 90,71 | 0 | 0 | 0 | 0 | 66,06 |
| 7 | 13,52 | 86,48 | 0 | 0 | 0 | 0 | 84,98 |
| 8 | 6,32 | 93,68 | 0 | 0 | 0 | 0 | 80,12 |
| 9 | 3,44 | 96,56 | 0 | 0 | 0 | 0 | 73,69 |

Abkürzungen MeOH = Methanol MTBE = Methyl-tert.-butylether

$\alpha$-Di = $\alpha$-Diisobutylen:2,4,4 Trimethyl-1-penten

$\beta$-Di = $\beta$-Diisobutylen:2,4,4 Trimethyl-2-penten

$\alpha$-Tri = $\alpha$-Triisobutylen:2,4,4,6,6 Pentamethyl-1-hepten

$\beta$-Tri = $\beta$-Triisobutylen:2,4,4,6,6 Pentamethyl-2-hepten 1-B = 1-Buten IB = Isobuten

Im Sinne der Erfindungsaufgabe läßt sich erkennen, daß das Sumpfprodukt frei von leichter siedenden Butenen ist.

Neben den vergleichenden kinetischen Studien und Untersuchungen in Füllkörperkolonnen, deren Ergebnisse vorstehend mitgeteilt wurden, wurden Langzeituntersuchungen zum Standzeitverhalten des erfindungsgemäßen Katalysators in einem Rohrreaktor bei wechselnden Katalysatorbelastungen durchgeführt.

Als Reaktor wurde ein V2A-Rohr von 770 mm Länge und 20 ml Durchmesser verwendet, welches zu Zweidrittel mit dem erfindungsgemäßen Katalysator in Form von Raschig-Ringen gefüllt war. Als $C_4$-Gemische wurden handelsübliche $C_4$-Schnitte der Raffinerie, 10 bzw. 49 Gew.-% Isobuten enthaltend, eingesetzt. Die Ergebnisse der Veretherung von Isopropanol bzw. Methanol mit Isobuten unter ausgewählten Betriebsparametern sind in den Tabellen 1 und 2 zusammengestellt.

Durch Vorschaltung einer Wasserwäsche und eines sogenannten Polizeifilters vor dem Eintritt des C4-Schnitts in die Reaktions-Trennkolonne können störende Bestandteile, die die Aktivität der Katalysator-Ringe vermindern, beispielsweise schwache Basen wie Amine oder Kationen wie Natrium wirksam entfernt werden. Je nach der Qualität des Methanol-Einsatzstromes empfiehlt sich evt. auch hier die Vorschaltung eines Polizeifilters. Mechanische Defekte an den Katalysator-Ringen waren nur in untergeordnetem Maße zu beobachten. Die Katalysator-Ringe aus sulfoniertem makroporösem Styrol-Polymerisat wurden als Hohlzylinder mit Abmessungen von ca. 6 mm Außen- und Innendurchmesser und 8 mm Höhe eingesetzt. Die Farbe der Hohlzylinder war weiß bis creme. Die Austauschkapazität wurde mit 4,44 mg H + /g Kontakt (trocken) nach der üblichen Methode (Fisher u. Kunin, Analyt. Chem. 27, 1955, 1191 - 1194) bestimmt. Das Quellvolumen von 23 ml trockenem Kontakt in wasser- oder methanol-feuchter Form betrug 50 ml, entsprechend einer Schüttdichte trocken von 0,275 g/ml bzw. 0,127 g/ml feucht. Der Vernetzungsgrad des eingesetzten Kontaktes entspricht dem nominellen Anteil an Divinylbenzol in dem monomeren Ausgangsgemisch. Die BET-Oberfläche des Kontaktes wurde nach der 1-Punkt-Methode mit $N_2$ Adsorption bestimmt.

Die erfindungsgegenständlichen Formkörper in Form von Hohlzylindern aus sulfoniertem Styrolpolymerisat wurden neben den Versuchsläufen zur Veretherung von verschiedenen i-Buten-Schnitten mit Isopropanol bzw. mit Methanol, deren Ergebnisse vorstehend mitgeteilt wurden, bei folgenden anderen Reaktionen mit gutem Erfolg erprobt, wobei die gleiche Apparatur wie bei Durchführung der vorgenannten Langzeitversuche benutzt wurde.

1. Hydratisierung von Propen mit Wasser (Tabelle 3)
2. Hydratisierung von i-Buten mit Wasser (Tabelle 4)
3. Veretherung von 2-Methyl-2-Buten mit Methanol (Tabelle 5)
4. Dimerisierung von $i-C_4$ (Tabelle 6)

Desweiteren wurden die erfindungsgemäßen Hohlzylinder aus sulfoniertem makroporösem Styrolpolymerisat mit Palladium dotiert und im Hinblick auf ihre Eignung als Katalysator für die hydrierende Veretherung getestet.

Die Belastung mit Palladium wurde, wie nachfolgend beschrieben, durchgeführt:

0,63 Pd $(NO_3)_2$ . $2H_2O$ wurden in 60 ml Wasser gelöst. Die Lösung war braungelb gefärbt. Hierzu wurden 100 ml $H_2O$-feuchte Styrolpolymerisat-Formkörper gegeben. Das Styrolpolymerisat war völlig von der Palladiumnitratlösung bedeckt. Nach 1 Tag Standzeit war die wäßrige Lösung wasserklar, was ein Zeichen für den Austausch des Palladiums war.

Als Einsätze wurden handelsübliches Methanol und Leichtbenzin aus einer Olefinanlage verwendet.

Nach Einfüllen der Formkörper in den vorhergehend bereits beschriebenen Rohrreaktor wurde das in den Raschig-Ringen enthaltene Restwasser mit Methanol verdrängt und das Palladium bei einer Temperatur von 70 °C und einem Druck von 11 bar mit 6 Normliter Wasserstoff in 150 ml Methanol pro Stunde reduziert. Nach 48 Stunden wurde Methanol durch das Reaktionseinsatzgemisch Benzin/Methanol ersetzt. Die weiteren Reaktionsbedingungen waren:

| | |
|---|---|
| Katalysatormenge: | 100 ml (gequollen in Wasser) |
| Temperatur: | 70 - 75 °C |
| Druck: | 10 - 11 bar |
| Feedeinsatz: | Crack-L-Destillat aus VC mit 10 % Methanol |
| Feedmenge: | 150 ml/h = 103 g/h |
| $H_2$-Menge: | 7 NL/h |
| Bi/$H_2$-Verhältnis: | 200 g/g |

Der Versuch hat gezeigt, daß die mit Palladium beladenen erfindungsgemäßen Formkörper sehr selektiv die in Pyrolyseleichtbenzin enthaltenen mehrfach ungesättigten Olefine (Diene) hydrieren und gleichzeitig die Veretherung tertiärer Monoolefine mit primären und sekundären Alkoholen katalysieren. Ergebnisse siehe Tabelle 7.

TABELLE 1

C$_4$-Schnitt Veretherung mit IPA

| Versuchs-Nr. | 1 | 2 | 3 | 4 | 5 | 6 | 7 | 8 | 9 |
|---|---|---|---|---|---|---|---|---|---|
| Kat.-Vol.gequollen(ml) | 160 | 160 | 160 | 160 | 160 | 160 | 160 | 160 | 160 |
| LHSV (ml Einsatz/ ml Katalysator) | 2,46 | 2,46 | 2,46 | 2,46 | 2,46 | 2,46 | 2,46 | 2,46 | 4,92 |
| Molverhaeltnis (Isobuten:Isopropanol) | 0,5 | 0,5 | 0,5 | 0,5 | 0,5 | 0,5 | 0,5 | 0,5 | 0,5 |
| Einsaetze | | | | | | | | | |
| C$_4$-Schnitt (ml/h) | 333 | 333 | 333 | 333 | 333 | 333 | 333 | 333 | 667 |
| IPA (ml/h) | 60 | 60 | 60 | 60 | 60 | 120 | 60 | 60 | 120 |
| Temperaturen, Druck | | | | | | | | | |
| Eingang (°C) | 53,2 | 53,8 | 54,2 | 58,6 | 58,6 | 58,7 | 58,6 | 58,7 | 58,7 |
| Ausgang (°C) | 55,2 | 56,4 | 57,2 | 59,0 | 59,2 | 59,2 | 59,2 | 59,4 | 59,5 |
| Druck (bar) | 11 | 11 | 11 | 11 | 11 | 11 | 11 | 11 | 11 |
| Ausbeute Isopropyl-tert.Butylether (Mol-%) | 56,0 | 56,5 | 56,4 | 55,6 | 54,5 | 51,8 | 54,4 | 54,8 | 46,3 |
| Laufzeit am Versuchsende (h) | 5 | 12 | 30 | 44 | 65 | 79 | 91 | 105 | 119 |

T A B E L L E   2

## Veretherung von i-Buten mit Methanol

| Versuchs-Nr. | 1 | 2 | 3 | 4 | 5 | 6 | 7 |
|---|---|---|---|---|---|---|---|
| Kat.-Volumen,gequollen (ml) | 200 | 200 | 200 | 200 | 200 | 200 | 200 |
| LHSV (ml Eins./ml Kat.) | 3,98 | 3,99 | 4,03 | 3,97 | 6,80 | 6,84 | 6,85 |
| Molverhaeltnis (Isobuten : Methanol) | 0,83 | 0,83 | 0,85 | 0,83 | 0,84 | 0,83 | 0,82 |
| Mengenstroeme | | | | | | | |
| $C_4$-Schnitt (g/h) | 370,6 | 371,0 | 375,6 | 369,9 | 633,3 | 636,3 | 635,2 |
| Methanol (g/h) | 124,3 | 124,8 | 125,1 | 123,5 | 209,4 | 214,6 | 216,5 |
| Temperaturen, Druck | | | | | | | |
| Eingang (°C) | 59,0 | 58,0 | 59,7 | 60,4 | 49,1 | 48,0 | 49,0 |
| Ausgang (°C) | 62,6 | 62,9 | 62,6 | 62,7 | 67,3 | 65,2 | 65,3 |
| Druck (bar) | 10 | 10 | 10 | 10 | 10 | 10 | 10 |
| Ausbeute MTBE (Mol-%) | 80,1 | 82,1 | 83,4 | 83,3 | 64,6 | 60,4 | 62,1 |
| Laufzeit bei Versuchsende (h) | 7 | 14 | 21 | 28 | 35 | 42 | 49 |

TABELLE 3

Hydratisierung von Propen mit Wasser

| Versuchs-Nr. | | 1 | 2 | 3 | 4 | 5 | 6 |
|---|---|---|---|---|---|---|---|
| Kat.-Volumen, gequollen (ml) | | 200 | 200 | 200 | 200 | 200 | 200 |
| LHSV | (ml Eins./ml Kat.) | 1 | 1 | 1 | 0,5 | 0,5 | 0,5 |
| Molverhaeltnis | ($H_2O/C_3$) | 10,3 | 9,2 | 9,8 | 10,2 | 10,4 | 10,3 |
| Mengenstroeme | | | | | | | |
| Propen | (g/h) | 32,6 | 32,1 | 32,2 | 15,9 | 15,8 | 15,7 |
| Wasser | (g/h) | 142,0 | 128,0 | 134,0 | 69,0 | 70,0 | 68,5 |
| Ausgang, stabilisiert | (g/h) | 144,2 | 131,5 | 139,9 | 73,2 | 74,4 | 73,1 |
| Temperaturen, Druck | | | | | | | |
| Eingang | (°C) | 120 | 131 | 142 | 139 | 139 | 138 |
| Ausgang | (°C) | 118 | 128 | 138 | 137 | 137 | 137 |
| Druck | (bar) | 80 | 80 | 80 | 80 | 80 | 80 |
| IPA-Gehalt des Reaktionsproduktes (Gew.-%) | | 2,2 | 3,8 | 6,0 | 8,2 | 8,5 | 8,9 |
| Umsatz Propen | (%) | 6,8 | 10,9 | 18,3 | 26,4 | 28,0 | 29,0 |
| Kontaktlaufzeit bei Versuchsende (h) | | 7 | 14 | 21 | 28 | 35 | 42 |

EP 0 417 407 B1

T A B E L L E   4

<u>Hydratisierung von i-Buten mit Wasser</u>

| Versuchs-Nr. | 7 | 8 | 9 | 10 | 11 | 12 |
|---|---|---|---|---|---|---|
| Kat.-Volumen, gequollen (ml) | 200 | 200 | 200 | 200 | 200 | 200 |
| LHSV (ml Einsatz/ml Katalysator) | 0,5 | 0,49 | 0,51 | 0,24 | 0,22 | 0,23 |
| Molverhaeltnis ($H_2O$/i-$C_4$) | 10,1 | 10,2 | 10,2 | 12,3 | 11,2 | 9,2 |
| Mengenstroeme | | | | | | |
| i-Buten (g/h) | 20,2 | 19,7 | 20,7 | 8,4 | 8,3 | 9,7 |
| Wasser (g/h) | 66,2 | 65,2 | 67,6 | 33,3 | 29,8 | 28,6 |
| Ausgang, stabilisiert (g/h) | 67,7 | 66,9 | 69,3 | 34,4 | 31,0 | 30,0 |
| Temperaturen, Druck | | | | | | |
| Eingang (°C) | 80,2 | 80,2 | 80,1 | 77,8 | 78,7 | 78,9 |
| Ausgang (°C) | 79,7 | 80,2 | 80,4 | 79,5 | 81,3 | 81,3 |
| Druck (bar) | 80 | 80 | 80 | 80 | 80 | 80 |
| TBA-Gehalt des Reaktionsproduktes (Gew.-%) | 3,0 | 3,3 | 3,0 | 4,1 | 5,2 | 6,1 |
| Umsatz i-Buten (%) | 7,6 | 8,5 | 7,6 | 12,7 | 14,7 | 14,3 |
| Kontaktlaufzeit bei Versuchsende (h) | 49 | 56 | 63 | 70 | 77 | 84 |

EP 0 417 407 B1

T A B E L L E   5

Veretherung von 2-Methyl-2-Buten mit Methanol

| Versuchs-Nr. | | 8 | 9 | 10 | 11 | 12 | 13 | 14 | 15 |
|---|---|---|---|---|---|---|---|---|---|
| Kat.-Volumen, gequollen | (ml) | 200 | 200 | 200 | 200 | 200 | 200 | 200 | 200 |
| LHSV (ml Einsatz : ml Katalysator) | | 4,0 | 4,0 | 4,0 | 4,1 | 2,0 | 2,0 | 2,0 | 2,0 |
| Molverhaeltnis (Meth.-But./MeOH) | | 0,84 | 0,92 | 0,91 | 0,92 | 0,90 | 0,89 | 0,87 | 0,87 |
| Mengenstroeme | | | | | | | | | |
| 2-Methyl-2-Buten | (g/h) | 364,5 | 374,5 | 375,3 | 379,3 | 185,7 | 185,2 | 184,8 | 184,2 |
| Methanol | (g/h) | 198,6 | 185,8 | 187,4 | 187,9 | 94,7 | 95,3 | 96,6 | 95,9 |
| Temperaturen, Druck | | | | | | | | | |
| Eingang | (°C) | 59,0 | 58,0 | 58,0 | 58,0 | 61,3 | 61,2 | 60,8 | 61,3 |
| Ausgang | (°C) | 62,6 | 61,5 | 61,9 | 61,9 | 59,9 | 59,8 | 59,7 | 59,8 |
| Druck | (bar) | 10 | 10 | 10 | 10 | 10 | 10 | 10 | 10 |
| Ausbeute TAME* (Mol-%) | | 26,9 | 24,3 | 25,0 | 23,7 | 37,3 | 37,3 | 37,4 | 37,5 |
| Kontaktlaufzeit bei Versuchsende (h) | | 140 | 147 | 154 | 161 | 168 | 175 | 182 | 189 |

*TAME = Tertiaer-amyl-Methylether

EP 0 417 407 B1

TABELLE 6

Dimerisierung von Isobuten

| Versuchs-Nr. | | 1 | 2 | 3 | 4 | 5 | 6 | 7 |
|---|---|---|---|---|---|---|---|---|
| Kat.-Volumen, gequollen (ml) | | 200 | 200 | 200 | 200 | 200 | 200 | 200 |
| LHSV (ml Einsatz : ml Katalysator) | | 1 | 1 | 1 | 1 | 1 | 1 | 2 |
| Einsatz | | | | | | | | |
| C$_4$-Gemisch | (g/h) | 118,5 | 117,7 | 114,3 | 113,7 | 116,7 | 116,7 | 233,0 |
| i-Buten *) | (g/h) | 57,8 | 57,4 | 55,8 | 55,9 | 56,9 | 54,8 | 109,5 |
| Temperaturen, Druck | | | | | | | | |
| Eingangstemperatur | (°C) | 73,8 | 74,4 | 72,2 | 72,5 | 82,8 | 82,6 | 90,1 |
| Druck | (bar) | 20,0 | 20,0 | 20,0 | 20,0 | 20,0 | 20,0 | 20,0 |
| Ausbeute Dimere | (Mol-%) | 92,9 | 94,4 | 95,8 | 93,5 | 93,5 | 94,3 | 96,3 |
| Kat.-Laufzeit | (h) | 197 | 205 | 213 | 221 | 245 | 253 | 261 |

*) Enthalten im C$_4$-Gemisch

EP 0 417 407 B1

TABELLE 7

__Veretherung unter hydrierenden Bedingungen__

| Komponente | | Einsatz | Reaktionsprodukt |
|---|---|---|---|
| Methanol | (Gew.%) | 10,5 | 6,8 |
| ≤ 2-Methylbutene | (Gew.%) | 17,4 | 12,8 |
| ≤ 2-Methylpentene | (Gew.%) | 5,7 | 4,6 |
| Cyclopentadien | (Gew.%) | 0,59 | < 0,005 |
| cis-Pentadien-1,3 | (Gew.%) | 0,12 | < 0,005 |
| trans-Pentadien-1,3 | (Gew.%) | 0,21 | < 0,005 |
| Methylbutadien | (Gew.%) | 0,11 | < 0,005 |
| TAME | (Gew.%) | – | 6,7 |
| 2-Methoxi-2-methylpentan | (Gew.%) | – | 1,5 |
| Sonstige sauerstoffhaltige Komponenten | (Gew.%) | – | 2,1 |

**Patentansprüche**

1. Verfahren zur Herstellung von Formkörpern aus makroporösem starksaurem oder basischem Ionenaustauscherharz in Gestalt von Füllkörpern wie Raschig-Ringen, Berl-Sätteln, Torus-Sätteln, Füllringen mit Steg oder Kreuzsteg, Pall-Ringen, sonstigen Hohlkörpern, Hohlkugeln u. dgl. mit einem Hohlraumanteil von 5 bis 95 Vol.-% der Makroform ohne Poren, einer BET-Oberfläche von 0,1 bis 1000 m$^2$/g, vorzugsweise 20 bis 60 m$^2$/g und einer Austauschkapazität von 0,05 bis 10, vorzugsweise 3 bis 6 meq/g, durch polymerisationsinitiierte Copolymerisation, insbesondere von Styrol und Divinylbenzol im Gewichtsverhältnis von 200 : 1 bis 1 : 8, vorzugsweise 20 : 1 bis 5 : 1 unter Zusatz von 2 bis 80 Gew.-%, vorzugsweise 10 bis 40 Gew.-% Porenbildner zu der Gesamtmischung, wobei diese Mischung in an sich zur Herstellung geformter Copolymerisate bekannter Weise einer formgebenden Maßnahme wie Formguß, Spritzguß oder Extrusion unterworfen wird und nach dem Aushärten des Formkörpers der Porenbildner z. B. durch Spülen entfernt wird.

2.  Verfahren nach Anspruch 1, dadurch gekennzeichnet, daß als Porenbildner Alkane wie n-Pentadecan eingesetzt werden.

3.  Verfahren nach Anspruch 1 oder 2, gekennzeichnet durch die Generierung oder Aufbringung saurer Zentren, insbesondere durch nachträgliche Säurebehandlung, z. B. mit Sulfonsäure.

4.  Formkörper herstellbar durch ein Verfahren nach einem der vorstehenden Ansprüche.

5.  Formkörper gemäß Anspruch 4, dotiert mit Metallen der 7. oder auch 8. Nebengruppe des Periodensystems, insbesondere Palladium, Ruthenium, Rhodium, in Mengen von 0,1 bis 100 g/l Ionenaustauscherharz.

6.  Formkörper gemäß Anspruch 4 oder 5 in Form von Raschig-Ringen mit einem Innendurchmesser von 0,5 bis 100 mm, vorzugsweise 4 bis 20 mm, einer Wandstärke von 0,1 bis 20 mm, vorzugsweise 0,5 bis 3 mm und einer Länge, die dem 0,1 - bis 20fachen des Innendurchmessers entspricht.

7.  Verwendung der Formkörper gemäß einem der Ansprüche 4 bis 6 als katalytisch aktive Füllkörper für die chemischen Reaktionen der Veretherung, Hydratisierung, Dimerisierung, Oligomerisierung, Veresterung, Hydrierung oder Alkylierung oder Kombinationen derselben.

## Claims

1.  Process for the production of moulded articles comprising macroporous strongly acidic or basic ion exchange resin in the form of fillers such as Raschig rings, Berl saddles, Torus saddles, filler rings with web or cross web, Pall rings, other hollow articles, hollow spheres and similar with a voids proportion of 5 to 95 vol.% of the macro-shape without pores, a BET surface of 0.1 to 1000 m$^2$/g, preferably 20 to 60 m$^2$/g and an exchange capacity of 0.05 to 10, preferably 3 to 6 meq/g, by polymerization-initiated copolymerization, particularly of styrene and divinyl benzene in the weight ratio from 200:1 to 1:8, preferably 20:1 to 5:1 with the addition of 2 to 80 wt.%, preferably 10 to 40 wt.% pore formers to the total mixture, whereby this mixture is subjected to a shaping measure such as casting, injection moulding or extrusion in a manner known per se for the production of moulded copolymerisates, and the pore former is removed e.g. by rinsing after the moulded article has been cured.

2.  Process according to claim 1, characterized in that alkanes such as n-pentadecane are used as pore formers.

3.  Process according to claim 1 or 2, characterized by the generation or application of acid centres, in particular by further acid treatment, e.g. with sulphonic acid.

4.  Moulded articles producible by a process according to one of the previous claims.

5.  Moulded articles according to claim 4, doped with metals of the 7th or also 8th subgroup of the periodic table system, particularly palladium, ruthenium, rhodium, in quantities of 0.1 to 100 g/l ion exchange resin.

6.  Moulded articles according to claim 4 or 5 in the form of Raschig rings with an inside diameter of 0.5 to 100 mm, preferably 4 to 20 mm, a wall thickness of 0.1 to 20 mm, preferably 0.5 to 3 mm and a length which corresponds to 0.1 to 20 times the inside diameter.

**7.** Use of the moulded articles according to one of claims 4 to 6 as catalytically active fillers for the chemical reactions of etherification, hydration, dimerisation, oligomerization, esterification, hydrogenation or alkylation or combinations of same.

**Revendications**

**1.** Procédé de production de corps moulés en résine échangeuse d'ions fortement acide ou basique, macroporeuse, sous forme de corps de remplissage comme des anneaux Raschig, des selles de Berl, des selles toriques, des anneaux de remplissage avec traverse ou entretoise, des anneaux Pall, d'autres corps creux, des billes creuses et semblables, avec un pourcentage d'espace creux de 5 à 95 % en volume de la macroforme sans pore, une surface BET de 0,1 à 1000 $m^2$/g, de préférence de 20 à 60 $m^2$/g et une capacité d'échange de 0,05 à 10, de préférence de 3 à 6 meq/g par copolymérisation mise en route par polymérisation, en particulier du styrène et du divinylbenzène dans un rapport pondéral de 200 : 1 à 1 : 8, de préférence de 20 : 1 à 5 :1, tout en ajoutant de 2 à 80 % en poids, de préférence de 10 à 40 %, en poids d'agent formateur de pores au mélange total, ce mélange étant soumis à une mesure de moulage d'une manière connue en soi pour la production de copolymères moulés comme la coulée dans un moule le moulage par injection ou l'extrusion et après durcissement du solide de moulage, l'agent de formation des pores est éliminé par exemple par rinçage.

**2.** Procédé selon la revendication 1, caractérisé en ce que l'on met en oeuvre comme agent de formation des pores des alcanes comme le n-pentadécane.

**3.** Procédé selon la revendication 1 ou 2, caractérisé par la génération ou l'application de centres acides, en particulier par traitement ultérieur par un acide, par exemple avec un acide sulfonique.

**4.** Corps de moulage que l'on peut produire par un procédé selon l'une des revendications précédentes.

**5.** Corps de moulage selon la revendication 4, doté avec des métaux des 7ème ou aussi 8ème sous-groupes du système périodique, en particulier le palladium, le ruthénium, le rhodium, en quantité allant de 0,1 à 100 g/l de résine échangeuse d'ions.

**6.** Corps de moulage selon la revendication 4 ou 5, sous forme d'anneaux de Raschig ayant un diamètre intérieur de 0,5 à 100 mm, de préférence de 4 à 20 mm, une épaisseur de paroi de 0,1 à 20 mm, de préférence de 0,5 à 3 mm et une longueur qui correspond à 0,1 à 20 fois le diamètre intérieur.

**7.** Utilisation des corps de moulage selon l'une des revendications 4 à 6 comme corps de remplissage, catalytiquement actifs, pour les réactions chimiques d'éthérification, d'hydratation, de dimérisation, d'oligomérisation, d'estérification, d'hydrogénation ou d'alcoylation ou des combinaisons de celles-ci.

Fig. 1

Fig. 2

EP 0 417 407 B1

Fig. 3

METHANOL

ISOBUTEN

1-BUTEN

Fig. 4

REST-BUTENE

METHANOL

BUTENE

KATALYSATORRINGE

KERAMIKRINGE

MTBE/METHANOL

EP 0 417 407 B1

Fig. 5